# EUROPÄISCHE PATENTSCHRIFT

(11) **EP 1 993 431 B1**
(45) Veröffentlichungstag und Bekanntmachung des Hinweises auf die Patenterteilung: **28.09.2016**
(21) Anmeldenummer: 07711845.3
(22) Anmeldetag: 08.03.2007
(51) Int. Cl.: A61B 3/113, G02B 27/00

(54) **VORRICHTUNGEN UND VERFAHREN ZUR DEFINIERTEN AUSRICHTUNG EINES AUGES**
DEVICES AND METHODS FOR DEFINED ORIENTATION OF AN EYE
DISPOSITIFS ET PROCÉDÉ PERMETTANT D'ALIGNER UN IL DE MANIÈRE DÉFINIE

(30) Priorität: 10.03.2006 DE 102006011624
(43) Veröffentlichungstag der Anmeldung: 26.11.2008
(73) Patentinhaber: Carl Zeiss Meditec AG, 07745 Jena (DE)
(72) Erfinder: BISCHOFF, Mark, 07749 Jena (DE); HANFT, Marco, 07743 Jena (DE); EBERT, Elke, 07743 Jena (DE); STOBRAWA, Gregor, 07743 Jena (DE)
(74) Vertreter: Beck, Bernard
(86) Internationale Anmeldenummer: PCT/EP2007/001984
(87) Internationale Veröffentlichungsnummer: WO 2007/104460

(56) Entgegenhaltungen:
- WO-A-02/053020
- WO-A-03/057023
- DE-A1- 10 151 314
- DE-A1- 19 812 050
- US-A- 6 145 990

## Beschreibung

Die vorliegende Erfindung betrifft eine Vorrichtung und ein entsprechendes Verfahren zur definierten Ausrichtung eines Auges zu diagnostischen oder therapeutischen Zwecken, wobei die Blickrichtung nicht zwingend mit der optischen Achse des Untersuchungs- oder Behandlungsgerätes überein zu stimmen braucht.

In der Augenheilkunde ist es bekannt, bei diagnostischen oder therapeutischen Verfahren das betreffende Auge des Patienten mit einer gewissen Genauigkeit bezüglich einer zur Durchführung des Verfahrens geeigneten Vorrichtung zu positionieren und zu orientieren. Dabei erfolgt die Orientierung und Positionierung des betreffenden Auges in der Regel auf bzw. entlang der optischen Achse des jeweiligen Gerätes.

Dazu wurde der Kopf des Patienten durch eine Kinnstütze und/oder Stirnstütze fixiert und im Anschluss daran das Gerät vom Bedienpersonal so positioniert, dass sich das Auge des Patienten annähernd mittig vor dem optischen System des Gerätes befand. Lag der Patient beispielsweise auf einer Liege, so wurde in der Regel die Liege mit dem Patienten bewegt und zum optischen System des Gerätes ausgerichtet. In jedem Fall erfolgt die Erfassung und Bewertung der Position des Auges des Patienten zunächst durch das Bedienpersonal, indem das Auge des Patienten direkt über ein optisches oder elektronisches Beobachtungssystem betrachtet und entsprechend zum Gerät, wie etwa einem ophthalmologischen Untersuchungsgerät oder einem Operationsmikroskop positioniert.

Im Anschluss an die Positionierung erfolgt die Orientierung des Patientenauges, d. h. die Ausrichtung dessen Blickrichtung, meist dadurch, dass dem Patienten ein Fixierlicht angeboten wird auf das sich das Auge ausrichtet. Das von der Vorrichtung bereitgestellte Fixierlicht wird in der Regel von einer separaten Lichtquelle erzeugt und auf der optischen Achse der Vorrichtung einblendet, so dass sich das Auge des Patienten entlang der optischen Achse der Vorrichtung ausrichtet und das Fixierlicht auf die Makula des Patienten fokussiert wird. Dabei werden nach dem bekannten Stand der Technik neben kontinuierlich leuchtenden auch blinkende Fixiermarken verwendet.

Insbesondere bei therapeutischen, ophthalmologischen Geräten wird die Orientierung des Patientenauges erfasst und ausgewertet, so dass eine festgestellte Fehlorientierung bei der therapeutischen Behandlung berücksichtigt werden kann. Bei dieser als Tracking bezeichneten automatischen Lageerfassung wird dabei weder das Patientenauge noch das Fixierlicht bewegt.

In der DE 102 54 369 A1 wird ein ophthalmologisches Gerät mit Eye-Tracker Einheit zur Vermessung von Strukturen mit Hilfe variabel strukturierter Beleuchtungsmuster beschrieben, mit dem Augenbewegungen durch Nachführung kompensiert werden können. Hierzu wird das Beleuchtungsmuster relativ zum Gerät entsprechend einer detektierten Augenbewegung bewegt, d. h. das Beleuchtungsmuster wird der Augenbewegung nachgeführt und scheint so, mit dem Auge "verbunden" zu sein. Die zur Detektion der Augenbewegung verwendete Eye-Tracker-Einheit ist mit der optischen Achse des Beobachtungssystems gekoppelt und liefert Signale die das Pupillenzentrum exakt definieren.

Damit das Beleuchtungsmuster nur für das Bedienpersonal und nicht für den Patienten sichtbar ist, wird es vorzugsweise von einer IR-Beleuchtung erzeugt und über eine entsprechende Kamera ausgewertet.

Die DE 103 14 944 A1 beschreibt eine Beleuchtungs- und Bestrahlungseinheit für ophthalmologische Geräte, zum Beleuchten/Bestrahlen des menschlichen Auges zum Zwecke der Beobachtung und/oder Behandlung. Das von der Beleuchtungsquelle erzeugte Licht wird über Mittel zur Erzeugung spezieller Beleuchtungsmuster, wie beispielsweise Filter, Blenden und/oder optoelektronische Lichtmodulatoren, und optische Mittel, wie Strahlteiler, halbdurchlässige Spiegel o. ä. in das Beobachtungssystem eingekoppelt. Die so erzeugten Beleuchtungsmuster werden von einer Projektionsoptik und dem Objektiv des ophthalmologischen Gerätes in das Patientenauge projiziert.

Obwohl die Lösung in erster Linie der Beleuchtung eines Auges dient, können erzeugte Beleuchtungsmuster mit Hilfe einer Eye-Tracker-Einheit relativ zum Gerät entsprechend einer detektierten Augenbewegung nachgeführt werden.

Auch bei dieser Lösung ist das Beleuchtungsmuster nur für das Bedienpersonal und nicht für den Patienten sichtbar und wird der detektierten Augenbewegung nachgeführt.

Mit dem in der DE 103 59 239 A1 beschriebenen Verfahren zur Darstellung einer Fixiermarke für ophthalmologische Behandlungsgeräte sollen ungewollte Augenbewegungen eines zu behandelnden Auges verhindert oder zumindest minimiert werden. Dazu wird die Fixiermarke in das Blickfeld des Patienten projiziert, so dass dieser das zu behandelnde Auge durch foveale Fokussierung auf die Fixiermarke ausrichtet. In einer besonderen Ausgestaltung wird die Fixiermarke so bewegt, dass der Patient der Bewegung folgen kann.

Allerdings kann die Fixiermarke auch zur gezielten Positionierung des Auges verwendet werden, wobei die Fixiermarke in das Blickfeld des zu behandelnden Auges projiziert wird, so dass der Patient das zu behandelnde Auge durch foveale Fokussierung auf die Fixiermarke ausrichtet. Die Fixiermarke wird dann so bewegt, dass der Patient der Bewegung folgen kann.

Im Gegensatz zu den bisher genannten Schriften wird in der DE 41 08 435 eine Anordnung zur Fixationskontrolle beschrieben, die zwar vorzugsweise für Geräte zur Gesichtsfelduntersuchung, aber prinzipiell auch für andere ophthalmologische Untersuchungsgeräte anwendbar ist. Dabei sind Mittel zur Drehung einer strukturierten Fixiermarke um eine zentrale Achse vorgesehen, um einen funktionellen und reproduzierbaren Fixationsanreiz zu schaffen. Die Fixiermarke soll dabei nur bei fovealer Fixation des Probanden eine von ihm identifizierbare Struktur und Vorzugsrichtung aufweisen. Dies wird dadurch erreicht, dass die Fixiermarke beispielsweise als ein, während der Drehung diskrete Richtungsorientierungen annehmender Landoltring ausgebildet ist. Ein Malteserkreuz-Getriebe erzeugt aus einer gleichmäßigen Drehbewegung die diskreten Richtungsorientierungen der Fixiermarke in 90°-Drehungen mit einer Ruhephase. Der Proband hat während der Untersuchung den Schlitz des Landoltringes ständig zu verfolgen, was er nur bei entsprechender fovealer Fixation realisieren kann.

Obwohl bei dieser Lösung die Fixiermarke für den Patienten sichtbar ist kann die Fixiermarke nicht zur gezielten Positionierung des Auges verwendet werden, da die Fixiermarke mit Ausnahme einer kurzen Ruhephase ständig bewegt wird und sich damit die Blickrichtung des zu untersuchenden bzw. zu behandelnden Auges verändert. Darüber hinaus gibt es bei dieser Lösung nur die Möglichkeit die Fixiermarke mit einer gewissen Variation (Rotation) nur in einer vorgegebenen Richtung erscheinen zu lassen..

Die WO 03/057023 und DE 198 12 050 A1 beschreiben weitere ophthalmologische Untersuchangen mittels Lichtmarken zur Fixierung des Anges.

Der vorliegenden Erfindung liegt die Aufgabe zugrunde, eine Lösung zu entwickeln, mit der ein zu untersuchendes und/oder zu behandelndes Auge ausgerichtet werden kann, um eine definierte Lage zur optischen Achse des ophthalmologischen Gerätes einzunehmen.

Erfindungsgemäß wird die Aufgabe durch die Merkmale der unabhängigen Ansprüche gelöst. Bevorzugte Weiterbildungen und Ausgestaltungen sind Gegenstand der abhängigen Ansprüche.

Die erfindungsgemäße Lösung zur Ausrichtung eines Auges zu diagnostischen und/oder therapeutischen Zwecken besteht aus einer Fixierlichtquelle zur Erzeugung einer positionierbaren Fixiermarke, einer Einrichtung zur Einkopplung dieser in einen Strahlengang eines ophthalmologischen Gerätes, über dessen Abbildungsoptik die Fixiermarke in das zu untersuchende und/oder zu behandelnde Auge projiziert wird. Das Abbild der Fixiermarke ist dabei in verschiedenen Abständen und Winkellagen zur optischen Achse des ophthalmologischen Gerätes darstellbar, damit sich das zu untersuchende und/oder zu behandelnde Auge durch foveale Fokussierung auf diese Fixiermarke ausrichtet und eine definierte Lage zur optischen Achse des ophthalmologischen Gerätes einnimmt.

Die vorgeschlagene technische Lösung ist grundsätzlich in allen, zu diagnostischen und/oder therapeutischen Zwecken eingesetzten ophthalmologischen Geräte anwendbar. Die Ausrichtung in eine definierte Lage zur optischen Achse des ophthalmologischen Gerätes ist allerdings insbesondere zur Beobachtung und/oder Behandlung der vorderen Augenabschnitte von Bedeutung.

Bei der Lösung zur Ausrichtung eines Auges zu diagnostischen und/oder therapeutischen Zwecken wird dem Auge des Patienten eine sichtbare Fixiermarke angeboten auf die er sein Auge durch foveale Fokussierung ausrichtet. Zur erneuten Ausrichtung wird die Fixiermarke entsprechend verschoben, wobei stets der Bezug zur optischen Achse des ophthalmologischen Gerätes berücksichtigt wird.

Die Erfindung wird nachfolgend anhand von Ausführungsbeispielen näher beschrieben. Dazu zeigen
- Figur 1:: eine aus einer separaten Lichtquelle mit einem vorgelagerten Scanspiegel bestehende Fixierlichtquelle,
- Figur 2:: eine aus einem Array bestehende Fixierlichtquelle,
- Figur 3:: eine Anordnung zur Einkopplung der Fixiermarke in den Mitbeobachtungsstrahlengang und
- Figur 4:: ein zu untersuchendes und/oder zu behandelndes Auge.

Die erfindungsgemäße Vorrichtung zur Ausrichtung eines Auges zu diagnostischen und/oder therapeutischen Zwecken besteht aus einer Fixierlichtquelle zur Erzeugung einer positionierbaren Fixiermarke und einer Einrichtung zur Einkopplung dieser in einen Strahlengang eines ophthalmologischen Gerätes, über dessen Abbildungsoptik die Fixiermarke auf das zu untersuchende und/oder zu behandelnde Auge projiziert wird, und einer Steuereinheit mit der das Abbild der Fixiermarke in verschiedenen Abständen und Winkellagen zur optischen Achse des ophthalmologischen Gerätes darstellbar ist, damit sich das zu untersuchende und/oder zu behandelnde Auge durch foveale Fokussierung auf diese Fixiermarke ausrichtet und eine definierte Lage zur optischen Achse des ophthalmologischen Gerätes einnimmt.

In einer ersten Ausführungsform besteht die Fixierlichtquelle zur Erzeugung der positionierbaren Fixiermarke aus einer separaten Lichtquelle mit mindestens einem vorgelagertem Scanspiegel, wobei als separate Lichtquelle ein selbstleuchtendes Objekt oder dessen Abbild bzw. eine beleuchtete Blende oder deren Abbild Verwendung finden.

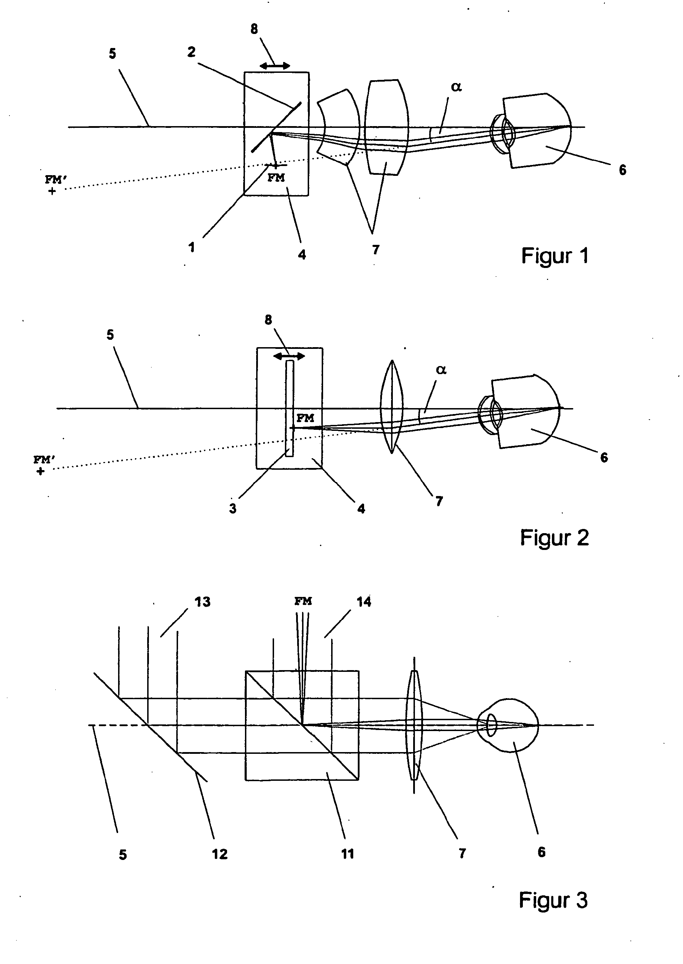

**Figur 1** zeigt dazu eine aus einer separaten Lichtquelle **1** mit einem vorgelagerten Scanspiegel **2** bestehende Fixierlichtquelle **4.** Die Abbildung verzichtet der Einfachheit halber auf die Darstellung einer Einrichtung zur Einkopplung der Fixiermarke **FM** in einen Strahlengang des ophthalmologischen Gerätes. Statt dessen wird die Fixierlichtquelle **4** nur in bezug auf die optische Achse **5** des ophthalmologischen Gerätes, sowie das zu untersuchende und/oder zu behandelnde Auge **6** dargestellt. Die von der Fixierlichtquelle **4** erzeugte positionierbare Fixiermarke **FM** wird über die Abbildungsoptik **7** des ophthalmologischen Gerätes in Richtung des zu untersuchenden und/oder zu behandelnden Auges **6** projiziert, dass der Patient dessen Abbild **FM'** scharf sieht. Die für das Erzeugen der Fixiermarke **FM** und/oder deren Abbild **FM'** in verschiedenen Abständen und Winkellagen zur optischen Achse **5** vorhandene Steuereinheit ist ebenfalls nicht dargestellt. Durch entsprechende Auslenkung des Scanspiegels **2** ändert sich der Winkel α, unter dem die Fixiermarke **FM** oder deren Abbild **FM'** sichtbar wird.

In einer besonderen Ausgestaltungsvariante zu dieser Lösung kann die separate Lichtquelle **1** so angeordnet werden, dass eine im ophthalmologischen Gerät bereits vorhandene Scaneinheit zur Erzeugung eines Abbildes der Fixiermarke **FM** in verschiedenen Abständen und Winkellagen genutzt werden kann. Insbesondere können dazu die in Therapiestrahlengängen vorhandenen scannenden Elemente benutzt werden, um das Auge für die therapeutische Behandlung auszurichten. Dies hat jedoch den Nachteil, dass die Fixiermarke während der therapeutischen Behandlung nicht mehr zur Ausrichtung des Auges genutzt werden kann, da eine gleichzeitige Bewegung mit den Therapiestrahlen erfolgt.

In einer zweiten Ausführungsform besteht die Fixierlichtquelle zur Erzeugung der positionierbaren Fixiermarke **FM** aus einem Display bzw. Array, auf welchem die Fixiermarke **FM** an beliebigen Orten dargestellt werden kann. Das Display bzw. Array ist als selbstleuchtende oder beleuchtete Einheit ausgeführt.

**Figur 2** zeigt dazu eine aus einem Array **3** bestehende Fixierlichtquelle **4.** Auch hier verzichtet die Abbildung auf die Darstellung einer Einrichtung zur Einkopplung der Fixiermarke in einen Strahlengang des ophthalmologischen Gerätes und stellt statt dessen die Fixierfichtquelle **4** nur in bezug auf die optische Achse **5** des ophthalmologischen Gerätes, sowie das zu untersuchende und/oder zu behandelnde Auge **6** dar. Die von der Fixierlichtquelle **4** erzeugte positionierbare Fixiermarke **FM** wird über die Abbildungsoptik **7** des ophthalmologischen Gerätes in Richtung des zu untersuchenden und/oder zu behandelnden Auges **6** projiziert, so dass der Patient dessen Abbild **FM'** scharf sieht. Die für das Erzeugen der Fixiermarke **FM** oder deren Abbild **FM'** in verschiedenen Abständen und Winkellagen zur optischen Achse **5** vorhandene Steuereinheit ist ebenfalls nicht dargestellt. Durch entsprechende Ansteuerung verschiedener Pixel des Arrays **3** lässt sich die Fixiermarke an verschiedenen Orten des Arrays **3** darstellen, wodurch sich der Winkel α, unter dem die Fixiermarke **FM** sichtbar wird, ändert.

Bei allen bisher erwähnten Ausgestaltungsvarianten besteht optional die Möglichkeit, bei der Abbildung der erzeugten, positionierbaren Fixiermarke **FM** über die Abbildungsoptik **7** des ophthalmologischen Gerätes individuelle Anforderungen an die axiale Bildlage zu berücksichtigen. Dies kann durch die Änderung der relativen Lage der erzeugten Fixiermarke zur Abbildungsoptik **7** des ophthalmologischen Gerätes (durch den Pfeil **8** dargestellt) realisiert werden.

Die Abstandsänderung bewirkt eine Änderung der Bildlage, bzw. der Divergenz des Strahlenbündels zwischen ophthalmologischem Gerät und Auge **6.** Mit dieser Funktionalität können verschiedene Aufgaben, wie beispielsweise:
- Dioptrienausgleich bei fehlsichtigen Patienten,
- Ausgleich einer Brechzahländerung zwischen ophthalmologischem Gerät und Auge (z. B. wässrige Lösung statt Luft),
- Anpassung einer Brechkraftänderung bei Verwendung eines Kontaktglases o. ä.
gelöst werden.

In einer besonderen Ausgestaltung ist es denkbar, dass basierend auf dieser Funktionalität (Divergenzänderung / Dioptrienausgleich) der Patient mittels eines Bedienelementes, wie z. B. einem Joystik, die finale Fokussierung der Fixiermarke selbst vornimmt.

Neben diesen genannten Effekten wäre es dadurch beispielsweise auch möglich, den Abbildungsmaßstab des Systems zu verändern.

In einer besonders vorteilhaften Ausgestaltung ist die Einrichtung zur Einkopplung der positionierbaren Fixiermarke **FM** am Mitbeobachtungsstrahlengang des ophthalmologischen Gerätes angeordnet.

Dazu zeigt **Figur 3** eine Anordnung zur Einkopplung der Fixiermarke in den Mitbeobachtungsstrahlengang eines opthalmoloigischen Gerätes.

Wie bereits beschrieben kann der, in einem Therapiestrahlengang **13** vorhandene Scanspiegel **12** benutzt werden, um mit der Fixiermarke **FM** das Auge **6** für die therapeutische Behandlung in bezug auf die optische Achse **5** auszurichten. Während der therapeutischen Behandlung ist die Fixiermarke **FM** allerdings nicht mehr zur Ausrichtung nutzbar. Um sie auch während der therapeutischen Behandlung zur Ausrichtung nutzen zu können, erfolgt die Einkopplung der positionierbaren Fixiermarke **FM** in den Mitbeobachtungsstrahlengang **14**.Dazu wird im Strahlengang des ophthalmologischen Gerätes, vor der Abbildungsoptik 7 ein zusätzlicher Strahlteiler **11** angeordnet. Dadurch ist es möglich die Fixiermarke **FM** auch während der therapeutischen Behandlung zur Ausrichtung des Auges **6** zu nutzen.

Mit der Steuereinheit wird das Abbild der Fixiermarke **FM** so manipuliert, dass es in verschiedenen Abständen und Winkellagen zur optischen Achse des ophthalmologischen Gerätes erscheint, damit sich das zu untersuchende und/oder zu behandelnde Auge durch foveale Fokussierung auf diese Fixiermarke **FM** ausrichtet und eine definierte Lage zur optischen Achse des ophthalmologischen Gerätes einnimmt.

Während dies in der ersten Ausgestaltung, bei der die Fixierlichtquelle aus einer separaten Lichtquelle mit vorgelagertem Scanspiegel besteht, durch eine entsprechende Auslenkung des Scanspiegels erfolgt, wird in der zweiten Ausführungsform, bei der die Fixierlichtquelle aus einem Display bzw. Array besteht, die Fixiermarke durch entsprechende Ansteuerung an beliebigen Orten dargestellt. Das zu untersuchende und/oder zu behandelnde Auge richtet sich durch foveale Fokussierung auf diese Fixiermarke aus und nimmt eine definierte Lage zur optischen Achse des ophthalmologischen Gerätes ein. Die dafür erforderliche Projektionsrichtung bzw. der Ort für die Darstellung des Abbildes der Fixiermarke wird von der Steuereinheit zuvor berechnet.

In einer anderen Ausgestaltung ist die Steuereinheit zur Manipulation des Abbildes der Fixiermarke mit einer Detektoreinheit verbunden, die die Ausrichtung des zu untersuchenden und/oder zu behandelnden Auges kontrolliert. Dadurch wird es möglich, die Einstellung der gewünschten Ausrichtung des Auges iterativ durchzuführen, d. h. die Ausrichtung wird kontrolliert und bei Bedarf durch Annäherung korrigiert (geschlossener Regelkreis).

Bei dem erfindungsgemäßen Verfahren zur Ausrichtung eines Auges zu diagnostischen und/oder therapeutischen Zwecken wird die von einer Fixierlichtquelle erzeugte, positionierbare Fixiermarke in einen Strahlengang des ophthalmologischen Gerätes eingekoppelt und über dessen Abbildungsoptik in Richtung des zu untersuchenden und/oder zu behandelnden Auges projiziert, wobei ein Abbild der Fixiermarke in verschiedenen Abständen und Winkellagen zur optischen Achse des ophthalmologischen Gerätes darstellbar ist, sich das zu untersuchende und/oder zu behandelnde Auge durch foveale Fokussierung auf diese Fixiermarke ausrichtet und eine definierte Lage zur optischen Achse des ophthalmologischen Gerätes einnimmt.

Wie bereits zuvor beschrieben, kann die positionierbare Fixiermarke von einer Fixierlichtquelle in Form einer separaten Lichtquelle mit vorgelagertem Scanspiegel erzeugt werden, wobei als separate Lichtquelle ein selbstleuchtendes Objekt oder dessen Abbild bzw. eine beleuchtete Blende oder deren Abbild Verwendung finden. Oder die positionierbare Fixiermarke wird von einem Display bzw. Array erzeugt, indem die Fixiermarke an beliebigen Orten darstellbar ist, wobei als Display bzw. Array eine selbstleuchtende oder beleuchtete Einheit Verwendung findet.

In einer vorteilhaften Ausgestaltung erfolgt die Einkopplung der positionierbaren Fixiermarke in den Mitbeobachtungsstrahlengang des ophthalmologischen Gerätes. Auch dies wurde bereits beschrieben.

Um das zu untersuchende und/oder zu behandelnde Auge durch foveale Fokussierung auf diese Fixiermarke auszurichten und in eine definierte Lage zur optischen Achse des ophthalmologischen Gerätes zu bringen, wird von einer vorhandenen Steuereinheit ein Abbild der Fixiermarke in verschiedenen Abständen und Winkellagen zur optischen Achse des ophthalmologischen Gerätes erzeugt.

Von der Steuereinheit wird dafür der erforderliche Ort der Darstellung des Abbildes der Fixiermarke Steuereinheit berechnet.

In einer weiteren Ausgestaltung ist die Steuereinheit mit einer Detektoreinheit verbunden, die die Ausrichtung des Auges kontrolliert, um die gewünschte Ausrichtung iterativ zu erreichen, wobei die Fixiermarke für die Detektoreinheit nicht sichtbar sein muss.

Mit Hilfe der Detektoreinheit wird die Lage eines charakteristischen Punktes zu einer Zielkoordinate festgestellt, wobei als charakteristisch solche Punkte verstanden werden, die durch bestimmte optische Merkmale geeignet sind, von ihrer Umgebung unterschieden werden zu können. Beispielsweise wird hierzu die Pupillenmitte genutzt. Als Detektoreinheit können hierbei vorhandene Beobachtungs- und/oder Abbildungseinheiten der ophthalmologischen Geräte genutzt werden. Besonders vorteilhaft ist hierbei die Verwendung einer Kamera-Einheit, die das zu untersuchende und/oder zu behandelnde Auge, inklusive des charakteristischen Punktes aufnimmt. Das elektronische Abbild des Auges, inklusive des Abbildes der Fixiermarke und die Zielkoordinate, die beispielsweise der optischen Achse **5** des ophthalmologischen Gerätes entsprechen kann, sind die Ausgangsgrößen für die Lagebewertung und die Ermittlung der Korrekturkoordinaten.

Ist die Lageabweichung (Δx; Δy) festgestellt (manuell oder automatisch), so werden daraus Steuersignale abgeleitet und der Fixierlichtquelle zugeführt und die Lage der Fixiermarke oder dessen Abbild korrigiert. Das Patientenauge folgt dieser Bewegung und ändert so seine Lage zum ophthalmologischen Gerät. Nach der Bewegung des Patientenauges kann dessen Lage zur Zielkoordinate erneut kontrolliert und gegebenenfalls korrigiert werden. Es ist auf diese Weise eine geschlossene Regelschleife eingerichtet.

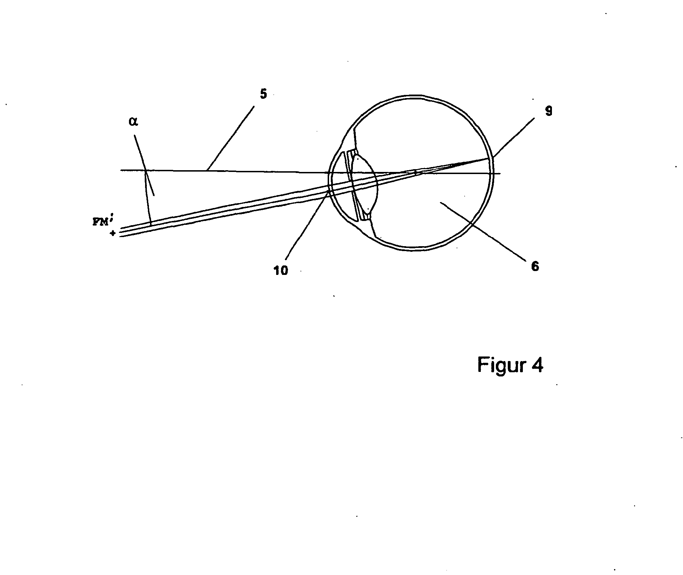

**Figur 4** zeigt zur näheren Erläuterung ein zu untersuchendes und/oder zu behandelndes Auge **6** mit dem Strahlengang der Fixiermarke **FM.**

Zu der Anwendung des ophthalmolgischen Gerätes (diagnostisch oder therapeutisch) muss das Auge **6** in bezug auf die optische Achse **5** des ophthalmologischen Gerätes ausgerichtet werden.

Von der Fixierlichtquelle **4** wird dem Auge **6** eine sichtbare Fixiermarke **FM** unter verschiedenen Winkeln α zur optischen Achse **5** angeboten auf die sich das Auge **6** durch foveale Fokussierung ausrichtet, indem sich das Auge **6** in der Augenhöhle so bewegt, dass die Fixiermarke **FM** in die Makula **9** scharf abgebildet wird.

Mit Hilfe der Detektoreinheit wird die Lage eines charakteristischen Punktes, wie beispielsweise die Pupillenmitte **10,** zu einer Zielkoordinate **ZK** festgestellt. Bei einer iterativen Annäherung liegt die Zielkoordinate **ZK** anfangs auf der optischen Achse **5.** Die sichtbare Fixiermarke **FM** wird nun unter einem Winkeln α zur optischen Achse **5** angeboten.

Erfolgt hierbei die Bilderfassung und Bildauswertung beispielsweise computergestützt, so kann die Ausrichtung des zu untersuchenden und/oder zu behandelnden Auges und dessen Positionierung in eine definierte Lage zur optischen Achse des ophthalmologischen Gerätes automatisch erfolgen.

Mit der erfindungsgemäßen Lösung ist die Ausrichtung eines Auges zu diagnostischen und/oder therapeutischen Zwecken auf einfache Art möglich.

Die meisten aus dem Stand der Technik bekannten Lösungen gehen implizit davon aus, dass Position und Orientierung des Patientenauges immer als weitgehend unabhängige Parameter optimiert werden müssen, um eine korrekte Bildgebung bei Diagnoseverfahren oder eine korrekte Behandlung bei Therapieverfahren zu ermöglichen. Dies ist zwar für Anwendungen im hinteren Augenabschnitt oft der Fall, im vorderen Augenabschnitt aber meist nicht. Insbesondere, wenn eine Beobachtung oder Bearbeitung der Komea erfolgt, ist meist nur die genaue Position der Kornea im Raum entscheidend.

Im Gegensatz zu den bekannten Lösungen geht die vorgeschlagene technische Lösung davon aus, dass die Drehbewegung des Auges in der Augenhöhle, die bei Blickrichtungsänderung erfolgt auch zur Positionsänderung der Kornea verwendet wird.

Dazu wird eine positionierbare Fixiermarke definiert bewegt und in Richtung des zu untersuchenden und/oder zu behandelnden Auges projiziert, welches sich durch foveale Fokussierung auf diese Fixiermarke ausrichtet und eine definierte Lage zur optischen Achse des ophthalmologischen Gerätes einnimmt.

Besonders vorteilhaft wirkt sich bei der vorgeschlagenen Lösung aus, dass damit das zu untersuchende und/oder zu behandelnde Auge sowohl in Richtung der optischen Achse des ophthalmologischen Gerätes, als auch in jede andere Richtung ausgerichtet werden kann.

## Patentansprüche

1. Vorrichtungen zur Ausrichtung eines Auges zu diagnostischen und/oder therapeutischen Zwecken, bestehend aus einer Fixierlichtquelle (4) zur Erzeugung einer positionierbaren Fixiermarke (FM), einer Einrichtung zur Einkopplung dieser in einen Strahlengang eines ophthalmologischen Gerätes, über dessen Abbildungsoptik (7) die Fixiermarke (FM) in Richtung des zu untersuchenden und/oder zu behandelnden Auges (6) projiziert wird, und einer Steuereinheit mit der das Abbild (FM') der Fixiermarke in verschiedenen Abständen und Winkellagen zur optischen Achse (5) des ophthalmologischen Gerätes darstellbar ist, damit sich das zu untersuchende und/oder zu behandelnde Auge (6) durch foveale Fokussierung auf diese Fixiermarke (FM) ausrichtet und eine definierte Lage zur optischen Achse (5) des ophthalmologischen Gerätes einnimmt, ***dadurch gekennzeichnet, dass die Fixierlichtquelle (4) so ausgebildet ist, dass der Abstand der erzeugten Fixiermarke (FM) zur Abbildungsoptik (7) veränderbar ist, um das Abbild (FM') der Fixiermarke In verschiedenen Fokusebenen entlang der optischen Achse (5) darzustellen.***

2. Vorrichtungen nach Anspruch 1, bei der die Fixierlichtquelle (4) zur Erzeugung der positionierbaren Fixiermarke (FM) aus einer separaten Lichtquelle (1) mit mindestens einem vorgelagertem Scanspiegel (2) besteht.

3. Vorrichtungen nach Anspruch 2, bei der als separate Lichtquelle (1) ein selbstleuchtendes Objekt oder dessen Abbild bzw. eine beleuchtete Blende oder deren Abbild Verwendung finden.

4. Vorrichtungen nach Anspruch 1, bei der die Fixierlichtquelle (4) zur Erzeugung der positionierbaren Fixiermarke (FM) aus einem Display bzw. Array (3) besteht, auf welchem die Fixiermarke (FM) an beliebigen Orten dargestellt werden kann.

5. Vorrichtungen nach Anspruch 4, bei der das Display bzw. Array (3) als selbstleuchtende oder beleuchtete Einheit ausgeführt ist.

6. Vorrichtungen nach mindestens einem der vorgenannten Ansprüche, bei der die Einrichtung zur Einkopplung der positionierbaren Fixiermarke (FM) am Mitbeobachtungsstrahlengang des ophthalmologischen Gerätes angeordnet ist.

7. Vorrichtungen nach Anspruch 7, bei dem die Steuereinheit mit einer Detektoreinheit verbunden ist, die die Ausrichtung des zu untersuchenden und/oder zu behandelnden Auges (6) kontrolliert.

8. Verfahren zur Ausrichtung eines Auges zu diagnostischen und/oder therapeutischen Zwecken, bei dem die von einer Fixierlichtquelle (4) erzeugte, positionierbare Fixiermarke (FM) in einen Strahlengang des ophthalmologischen Gerätes eingekoppelt und über dessen Abbildungsoptik (7) in Richtung des zu untersuchenden und/oder zu behandelnden Auges (6) projiziert wird, wobei ein Abbild (FM') der Fixiermarke in verschiedenen Abständen und Winkellagen zur optischen Achse (5) des ophthalmologischen Gerätes darstellbar ist, sich das zu untersuchende und/oder zu behandelnde Auge (6) durch foveale Fokussierung auf diese Fixiermarke ausrichtet und eine definierte Lage zur optischen Achse (5) des ophthalmologischen Gerätes einnimmt, ***dadurch gekennzeichnet, dass der Abstand der von der Fixierlichtquelle (4) erzeugten Fixiermarke (FM) zur Abbildungsoptik (7) veränderbar ist, um das Abbild (FM') der Fixiermarke in verschiedenen Fokusebenen entlang der optischen Achse (5) darzustellen.***

9. Verfahren nach Anspruch 9, bei dem die positionierbare Fixiermarke (FM) von einer separaten Lichtquelle (1) mit vorgelagertem Scanspiegel (2) erzeugt wird.

10. Verfahren nach Anspruch 9 und 10, bei dem als separate Lichtquelle (1) ein selbstleuchtendes Objekt oder dessen Abbild bzw. eine beleuchtete Blende oder deren Abbild Verwendung finden.

11. Verfahren nach Anspruch 9, bei dem die positionierbare Fixiermarke (FM) von einem Display bzw. Array (3) erzeugt wird, indem die Fixiermarke (FM) an beliebigen Orten darstellbar ist.

12. Verfahren nach Anspruch 9 und 12, bei dem als Display bzw. Array (3) eine selbstleuchtende oder beleuchtete Einheit Verwendung findet.

13. Verfahren nach mindestens einem der vorgenannten Ansprüche, bei dem die positionierbare Fixiermarke (FM) in den Mitbeobachtungsstrahlengang des ophthalmologischen Gerätes eingekoppelt wird.

14. Verfahren nach mindestens einem der vorgenannten Ansprüche, bei dem zur Darstellung des Abbildes (FM') der Fixiermarke in verschiedenen Abständen und Winkellagen zur optischen Achse (5) des ophthalmologischen Gerätes eine Steuereinheit vorhanden ist.

15. Verfahren nach mindestens einem der vorgenannten Ansprüche, bei dem der Ort der Darstellung des Abbildes (FM') der Fixiermarke von der Steuereinheit berechnet wird.

16. Verfahren nach mindestens einem der vorgenannten Ansprüche, bei dem die Steuereinheit mit einer Detektoreinheit verbunden ist, die die Ausrichtung Lage der Fixiermarke (FM) auf dem Auge (6) kontrolliert, um die gewünschte Ausrichtung iterativ zu erreichen.

## Claims

1. Systems for aligning an eye for diagnostic and/or therapeutic purposes, consisting of a fixation light source (4) for generating a positionable fixation mark (FM), of an apparatus for coupling the latter into a beam path of an ophthalmic device, with the fixation mark (FM) being projected in the direction of the eye (6) to be examined and/or to be treated by way of the imaging optics (7) of the ophthalmic device, and of a control unit, with which the image (FM') of the fixation mark is representable at different distances and angular positions with respect to the optical axis (5) of the of ophthalmic device, so that the eye (6) to be examined and/or to be treated aligns itself by way of foveal focusing on this fixation mark (FM) and assumes a defined position with respect to the optical axis (5) of the ophthalmic device, **characterized in that** the fixation light source (4) is configured such that the distance of the generated fixation mark (FM) with respect to the imaging optics (7) is variable to represent the image (FM') of the fixation mark in different focal planes along the optical axis (5).

2. Systems according to Claim 1, in which the fixation light source (4) for generating the positionable fixation mark (FM) consists of a separate light source (1) having at least one scanning mirror (2) which is located upstream.

3. Systems according to Claim 2, in which a selfluminous object or the image thereof and/or an illuminated stop or the image thereof are used as the separate light source (1).

4. Systems according to Claim 1, in which the fixation light source (4) for generating the positionable fixation mark (FM) consists of a display or array (3) on which the fixation mark (FM) can be represented at different locations.

5. Systems according to Claim 4, in which the display or array (3) is configured as a self-luminous or illuminated unit.

6. Systems according to at least one of the preceding claims, in which the apparatus for coupling in the positionable fixation mark (FM) is arranged at the coobservation beam path of the ophthalmic device.

7. Systems according to Claim 7, in which the control unit is connected to a detector unit, which checks the alignment of the eye (6) to be examined and/or to be treated.

8. Method for aligning an eye for diagnostic and/or therapeutic purposes, in which the positionable fixation mark (FM) that is generated by a fixation light source (4) is coupled into a beam path of the ophthalmic device and is projected, by the imaging optics (7) of said ophthalmic device, in the direction of the eye (6) to be examined and/or treated, wherein an image (FM') of the fixation mark is representable at different distances and angular positions with respect to the optical axis (5) of the of ophthalmic device, the eye (6) to be examined and/or to be treated aligns itself by foveal focusing on said fixation mark and assumes a defined position with respect to the optical axis (5) of the ophthalmic device, **characterized in that** the distance of the fixation mark (FM) that is generated by the fixation light source (4) from the imaging optics (7) is variable to represent the image (FM') of the fixation mark in different focal planes along the optical axis (5).

9. Method according to Claim 9, in which the positionable fixation mark (FM) is generated by a separate light source (1) having a scanning mirror (2) located upstream.

10. Method according to Claim 9 and 10, in which a self-luminous object or the image thereof and/or an illuminated stop or the image thereof are used as the separate light source (1).

11. Method according to Claim 9, in which the positionable fixation mark (FM) is generated by a display or array (3) with the fixation mark (FM) being representable at any desired location.

12. Method according to Claim 9 and 12, in which a self-luminous or illuminated unit is used as the display or array (3).

13. Method according to at least one of the preceding claims, in which the positionable fixation mark (FM) is coupled into the co-observation beam path of the ophthalmic device.

14. Method according to at least one of the preceding claims, in which a control unit is present for representing the image (FM') of the fixation mark at different distances and angular positions with respect to the optical axis (5) of the ophthalmic device.

15. Method according to at least one of the preceding claims, in which the location of the representation of the image (FM') of the fixation mark is calculated by the control unit.

16. Method according to at least one of the preceding claims, in which the control unit is connected to a detector unit, which checks the alignment position of the fixation mark (FM) on the eye (6) to achieve the desired alignment iteratively.

## Revendications

1. Dispositifs d'alignement d'un oeil à des fins diagnostiques et/ou thérapeutiques, constitués d'une source lumineuse de fixation (4) servant à générer un repère de fixation (FM) positionnable, et d'un système destiné à injecter celle-ci sur un trajet optique d'un appareil ophtalmologique, par l'intermédiaire de l'optique d'imagerie (7) duquel la marque de fixation (FM) est projetée dans la direction de l'oeil (6) à examiner et/ou à traiter, et d'une unité de commande au moyen de laquelle l'image (FM') du repère de fixation peut être représentée à des distances et des positions angulaires différentes par rapport à l'axe optique (5) de l'appareil ophtalmologique, de manière à ce que l'oeil (6) à examiner et/ou à traiter s'aligne par focalisation fovéale sur ce repère de fixation (FM) et adopte une position définie par rapport à l'axe optique (5) de l'appareil ophtalmologique, **caractérisé en ce que** la source de lumineuse de fixation (4) est réalisée de manière à ce que la distance du repère de fixation (FM) généré par rapport à l'optique d'imagerie (7) puisse être modifiée afin de représenter l'image (FM') du repère de fixation dans des plans de focalisation différents le long de l'axe optique (5).

2. Dispositifs selon la revendication 1, dans lesquels la source lumineuse de fixation (4) est constituée d'une source lumineuse séparée (1) comportant au moins un miroir de balayage (2) situé en amont pour générer le repère de fixation (FM) positionable.

3. Dispositifs selon la revendication 2, dans lesquels un objet auto-luminescent ou son image ou un obturateur éclairé ou son image sont utilisés en tant que source lumineuse séparée (1).

4. Dispositifs selon la revendication 1, dans lesquels la source lumineuse de fixation (4) est constituée d'un afficheur ou d'un réseau (3) destiné à générer le repère de fixation positionable (FM), sur lequel le repère de fixation (FM) peut être représenté à des emplacements quelconques.

5. Dispositifs selon la revendication 4, dans lesquels l'afficheur ou le réseau (3) est réalisé sous la forme d'une unité auto-luminescente ou éclairée.

6. Dispositifs selon au moins l'une des revendications précédentes, dans lesquels le système d'injection du repère de fixation (FM) positionable est disposé sur le chemin optique d'observation simultanée de l'appareil ophtalmologique.

7. Dispositifs selon la revendication 7, dans lesquels l'unité de commande est reliée à une unité de détection qui contrôle l'alignement de l'oeil (6) à examiner et/ou à traiter.

8. Procédé d'alignement d'un oeil à des fins diagnostiques et/ou thérapeutiques, dans lequel le repère de fixation (FM) positionable généré par une source lumineuse de fixation (4) est injecté dans un chemin optique de l'appareil ophtalmologique et est projeté par l'intermédiaire de l'optique d'imagerie (7) dudit appareil dans la direction de l'oeil (6) à examiner et/ou à traiter, dans lequel une image (FM') du repère de fixation peut être représentée à des distances et des positions angulaires différentes par rapport à l'axe optique (5) de l'appareil ophtalmologique, l'oeil (6) à examiner et/ou à traiter s'aligne et adopte une position définie par rapport à l'axe optique (5) de l'appareil ophtalmologique par focalisation fovéale sur ledit repère de fixation, **caractérisé en ce que** la distance du repère de fixation (FM) généré par la source lumineuse de fixation (4) par rapport à l'optique d'imagerie (7) peut être modifiée afin de représenter l'image (FM') du repère de fixation dans des plans de focalisation différents le long de l'axe optique (5).

9. Procédé selon la revendication 9, dans lequel le repère de fixation (FM) positionable est généré par une source lumineuse séparée (1) au moyen d'un miroir de balayage (2) situé en amont.

10. Procédé selon les revendications 9 et 10, dans lequel un objet auto-luminescent ou son image ou un obturateur éclairé ou son image sont utilisés en tant que source lumineuse séparée (1).

11. Procédé selon la revendication 9, dans lequel le repère de fixation (FM) positionable est généré par un afficheur ou un réseau (3) en faisant en sorte que le repère de fixation (FM) puisse être représenté à des emplacements quelconques.

12. Procédé selon les revendications 9 et 12, dans lequel une unité auto-luminescente ou éclairée est utilisée en tant qu'afficheur ou réseau (3).

13. Procédé selon au moins l'une des revendications précédentes, dans lequel le repère de fixation (FM) positionable est injecté dans le chemin optique d'observation simultanée de l'appareil ophtalmologique.

14. Procédé selon au moins l'une des revendications précédentes, dans lequel une unité de commande est prévue pour représenter l'image (FM') du repère de fixation à des distances et des positions angulaires différentes par rapport à l'axe optique (5) de l'appareil ophtalmologique.

15. Procédé selon au moins l'une des revendications précédentes, dans lequel la position de représentation de l'image (FM') du repère de fixation est calculée par l'unité de commande.

16. Procédé selon au moins l'une des revendications précédentes, dans lequel l'unité de commande est reliée à une unité de détection qui contrôle la position d'alignement du repère de fixation (FM) sur l'oeil (6) afin d'atteindre de manière itérative l'alignement souhaité.
